# FASCICULE DE BREVET EUROPEEN

(11) **EP 2 926 369 B1**
(45) Date de publication et mention de la délivrance du brevet: **22.02.2017**
(21) Numéro de dépôt: 13811809.6
(22) Date de dépôt: 29.11.2013
(51) Int. Cl.: H01L 21/673, G01N 33/00

(54) **STATION ET PROCEDE DE MESURE DE LA CONTAMINATION EN PARTICULES D'UNE ENCEINTE DE TRANSPORT POUR LE CONVOYAGE ET LE STOCKAGE ATMOSPHERIQUE DE SUBSTRATS SEMI-CONDUCTEURS**
STATION UND VERFAHREN ZUR MESSUNG DER PARTIKELKONTAMINATION EINER TRANSPORTKAMMER ZUM FÖRDERN UND ATMOSPHÄRISCHEN LAGERN VON HALBLEITERSUBSTRATEN
STATION AND METHOD FOR MEASURING THE PARTICULATE CONTAMINATION OF A TRANSPORT CHAMBER FOR CONVEYING AND ATMOSPHERICALLY STORING SEMICONDUCTOR SUBSTRATES

(30) Priorité: 30.11.2012 FR 1261453
(43) Date de publication de la demande: 07.10.2015
(73) Titulaire: Pfeiffer Vacuum, 74000 Annecy (FR)
(72) Inventeur: THOVEX, Cindy, F-74220 La Clusaz (FR); BELLET, Bertrand, F-73000 Chambéry (FR); CHAPEL, Nicolas, F-74150 Sales (FR)
(74) Mandataire: Croonenbroek, Thomas Jakob
(86) Numéro de dépôt international: PCT/EP2013/075076
(87) Numéro de publication internationale: WO 2014/083152

(56) Documents cités:
- FR-A1- 2 930 675
- US-A1- 2002 029 791

## Description

La présente invention concerne une station de mesure de la contamination en particules d'une enceinte de transport pour le convoyage et le stockage atmosphérique de substrats semi-conducteurs tels que des plaquettes semi-conductrices (« wafers » en anglais) ou des photomasques. L'invention concerne également un procédé de mesure correspondant.

Les enceintes de transport déterminent un espace confiné sous pression atmosphérique, séparé de l'environnement extérieur, pour le transport et le stockage d'un ou de plusieurs substrats.

Dans l'industrie de fabrication de semi-conducteurs, ces enceintes permettent de transporter les substrats d'un équipement à l'autre ou de stocker les substrats entre deux étapes de fabrication. On distingue notamment les enceintes standardisées de transport et de stockage de plaquettes à ouverture latérale de type FOUP (« Front Opening Unified Pod » en anglais) ou FOSB (« Front Opening Shipping Box » en anglais), ou à ouverture par le bas de type SMIF Pod (« Standard Mechanical Interface Pod » en anglais), ou encore enceintes standardisées de transport et de stockage de photomasques de type RSP (« Reticle SMIF Pod » en anglais) ou MRP (« Multiple Reticule SMIF Pod »).

Ces enceintes de transport sont formées de matériaux tels que le polycarbonate, qui peuvent dans certains cas concentrer les contaminants et en particulier des contaminants organiques, aminés ou acides. En effet au cours de la fabrication des semi-conducteurs, les enceintes de transport sont manipulées, ce qui conduit à la formation de particules polluantes qui se logent dans les parois des enceintes de transport et les contaminent. Les particules collées sur les parois des enceintes de transport peuvent ensuite se décoller, retomber sur les substrats stockés dans ces enceintes et les rendre inutilisables. Ces contaminations peuvent être très néfastes pour les substrats. Il est donc nécessaire de nettoyer ces enceintes. On prévoit donc leur nettoyage régulier par lavage avec un liquide tel que l'eau pure. Ces étapes de nettoyage sont réalisées soit directement dans les usines de fabrication de substrat semi-conducteur, soit dans des entreprises spécialisées dans le nettoyage d'enceintes de transport atmosphérique.

Pour déterminer lorsqu'une enceinte nécessite un nettoyage, on connaît un procédé de mesure de la contamination en particules consistant à mesurer le nombre de particules déposées sur les parois des enceintes de transport à l'aide d'un détecteur de particules liquide. Ce procédé présente toutefois l'inconvénient d'être long et lourd à implémenter dans un processus industriel de fabrication de semi-conducteur. De plus, ce type de procédé n'est pas reproductible. En effet, la mesure obtenue est directement liée à l'entreprise spécialisée ayant été chargée de réaliser la mesure, ce qui ne permet pas la mise en place de contrôles standardisés. Par conséquent, certaines enceintes de transport exemptes de particules sont tout de même nettoyées, réduisant ainsi inutilement les cadences de fabrication, tandis que d'autres, polluées de particules, continuent à stocker et/ou transporter des substrats de semi-conducteurs avec des risques potentiels de contamination des substrats.

Les industriels prévoient donc de fréquents nettoyages préventifs de manière à ne pas impacter le niveau de défectivité des substrats.

Pour éviter cela, on connaît par exemple du document WO 2009/138637, un dispositif de mesure de la contamination en particules d'enceintes de transport, permettant de réaliser une mesure en temps réel, directement dans l'usine de fabrication. Le dispositif de mesure comporte une première chambre destinée à ôter la porte de l'enceinte et une deuxième chambre destinée à mesurer la contamination en particules de l'enveloppe rigide de l'enceinte. L'interface comporte une buse d'injection articulée pour diriger un jet de gaz contre les parois internes de l'enveloppe rigide de manière à détacher les particules pour les mesurer par un compteur de particules. Pour améliorer le décrochage des particules, il est prévu de pouvoir pulser le flux de gaz injecté.

Toutefois ce dispositif de mesure ne permet pas de contrôler le niveau de contamination en particules de la porte qui est le plus souvent la paroi de l'enceinte de transport la plus polluée.

Un des buts de la présente invention est donc de proposer une station et un procédé de mesure correspondant permettant de mesurer un niveau de contamination en particules de toutes les parois internes des enceintes de transport atmosphériques, y compris de la porte.

A cet effet, l'invention a pour objet une station de mesure de la contamination en particules d'une enceinte de transport pour le convoyage et le stockage atmosphérique de substrats semi-conducteurs, ladite enceinte de transport comprenant une enveloppe rigide munie d'une ouverture et une porte amovible permettant d'obturer l'ouverture, la station de mesure comportant :
- une chambre à environnement contrôlé comportant au moins un port de chargement susceptible de s'accoupler d'une part à l'enveloppe rigide et d'autre part à la porte de l'enceinte de transport, pour déplacer la porte dans la chambre à environnement contrôlé,
- un module de mesure comportant une unité de mesure des particules,
- une interface de mesure enveloppe configurée pour s'accoupler à l'enveloppe rigide accouplée à la chambre à environnement contrôlé à la place de la porte, en définissant un premier volume de mesure, ladite interface de mesure enveloppe comportant au moins une buse d'injection et un premier orifice de prélèvement raccordé à l'unité de mesure des particules,
caractérisée en ce que ledit module de mesure comporte:
- une interface en creux de mesure porte configurée pour s'accoupler à la porte en définissant un deuxième volume de mesure entre une face de mesure du module de mesure et la porte en vis-à-vis, ladite face de mesure présentant au moins une buse d'injection et un deuxième orifice de prélèvement raccordé à l'unité de mesure des particules.

L'interface en creux de mesure porte permet ainsi de définir un volume de mesure confiné dans lequel les buses d'injections soufflent pour décrocher les particules de la porte et dans lequel un deuxième orifice de prélèvement raccordé à l'unité de mesure des particules prélève l'échantillon gazeux au moment de la réalisation d'une mesure. Il est donc possible de mesurer la contamination en particules de la porte. On profite en outre de la forme en coquille de l'enveloppe périphérique de l'enceinte de transport pour définir un volume de mesure confiné, séparé de la chambre à environnement contrôlé.

L'interface en creux de mesure porte présente par exemple une forme générale de cadre.

La porte est par exemple susceptible d'être déplacée en direction de l'interface en creux de mesure porte.

Le module de mesure peut comporter un mécanisme de déplacement module configuré pour déplacer l'interface de mesure enveloppe entre une position de repos et une position de mesure dans l'enveloppe rigide accouplée.

Selon un exemple de réalisation, l'interface de mesure enveloppe et l'interface en creux de mesure porte sont agencées dos à dos. Par exemple, l'interface de mesure enveloppe est susceptible d'être translatée dans l'enveloppe rigide accouplée et la porte est susceptible d'être translatée en direction de l'interface en creux de mesure porte.

La ligne de prélèvement de l'unité de mesure des particules peut comporter un dispositif à vanne pour basculer sélectivement vers le premier ou le deuxième orifice de prélèvement. On utilise ainsi un seul compteur de particules pour mesurer à la fois le niveau de contamination en particules de la porte et les parois internes de l'enveloppe rigide, ce qui permet de réduire les coûts de la station de mesure et les frais de maintenance.

Selon un mode de réalisation, l'interface de mesure enveloppe comporte au moins deux buses d'injection configurées pour diriger un jet de gaz sur au moins deux endroits distincts de l'enveloppe rigide accouplée à la chambre à environnement contrôlé, les orientations respectives des buses d'injection étant fixes par rapport à l'enveloppe rigide accouplée.

Ainsi ce n'est plus la buse d'injection qui est mobile pour souffler à différentes zones sur les parois internes de l'enveloppe rigide comme dans l'art antérieur. Le soufflage est réalisé au travers des buses d'injections qui sont d'orientation fixe, pour décrocher les particules et réaliser une mesure sans pièce mobile dans le volume confiné de l'enveloppe rigide fermé par l'interface de mesure enveloppe. On assure ainsi un soufflage au plus proche des parois internes de l'enveloppe rigide, sans mouvement des buses d'injection au moment du soufflage.

Par ailleurs, la distance entre les sorties des buses d'injection et les parois interne de l'enveloppe rigide, est maîtrisée et peut être optimisée.

De plus, l'orientation des jets de gaz et la valeur du couple débit/pression des buses d'injection étant maîtrisés et déterminés une fois pour toutes, le décrochage des particules peut être reproduit à l'identique, assurant la reproductibilité des mesures.

La station de mesure peut comporter une unité de traitement configurée pour commander l'injection sélective du gaz dans les buses d'injection.

Selon un mode de réalisation, l'interface de mesure enveloppe comporte une tête de mesure faisant saillie d'une base de l'interface de mesure enveloppe. Par exemple, ladite tête de mesure présente une forme générale parallélépipédique et chacune des cinq faces de la tête de mesure faisant saillie de la base de l'interface de mesure enveloppe peut comporter au moins une buse d'injection. On peut ainsi commander sélectivement l'injection du gaz dans chacune des cinq faces de l'enveloppe rigide, et mesurer tour à tour la contamination de chacune des faces, ce qui permet de déterminer avec précision la provenance de la contamination et un état de propreté pour chaque paroi interne de l'enceinte.

Les buses d'injection peuvent comporter des injecteurs en matériau dur, tels que des injecteurs en rubis, saphir ou zircone, permettant de définir avec une très haute précision les dimensions de l'orifice d'injection, ce qui permet une bonne reproductibilité des mesures. De plus, les injecteurs en matériau dur sont inusables, ce qui évite les dérives de dimension dans le temps.

Les buses d'injection peuvent par exemple être configurées pour diriger un jet de gaz dans au moins deux directions perpendiculaires entre elles et perpendiculaires aux parois d'une enveloppe rigide d'enceinte de transport accouplée à la chambre à environnement contrôlé. Un jet de gaz confiné perpendiculairement à la paroi permet d'améliorer l'impact sur les parois pour détacher efficacement les particules adhérées aux parois internes de l'enveloppe rigide.

L'unité de mesure des particules peut comporter des moyens de nettoyage configurés pour injecter un gaz de purge dans la ligne de prélèvement.

Les actionneurs du mécanisme d'actionnement de porte et/ou du mécanisme de déplacement module peuvent être agencés dans la chambre à environnement contrôlé et la chambre à environnement contrôlé peut comporter une unité de filtrage à flux laminaire pour placer l'atmosphère interne de la chambre à environnement contrôlé sous flux laminaire d'air filtré de sorte que les éventuelles particules générées par les actionneurs soient entraînées hors de la station de mesure.

La station de mesure peut comporter une armoire électrique déportée latéralement de la chambre à environnement contrôlé, ladite armoire électrique logeant une pompe à vide de l'unité de mesure des particules, évitant ainsi la contamination de la chambre à environnement contrôlé par les différents composants logés dans l'armoire électrique.

Selon un mode de réalisation particulier, la chambre à environnement contrôlé comporte deux ports de chargement susceptibles de s'accoupler à une enceinte de transport respective. Le mécanisme de déplacement module est par exemple configuré pour déplacer l'interface de mesure enveloppe entre une position de repos et une position de mesure dans l'une ou l'autre des enveloppes rigides accouplées.

L'invention a aussi pour objet un procédé de mesure de la contamination en particules d'une enceinte de transport pour le convoyage et le stockage atmosphérique de substrats semi-conducteurs, mis en oeuvre dans une station de mesure telle que décrite précédemment, caractérisé en ce qu'il comporte :
- une étape dans laquelle le module de mesure s'accouple à l'enveloppe rigide en définissant un premier volume de mesure entre l'interface de mesure enveloppe et l'enveloppe rigide accouplée pour réaliser une mesure de contamination des parois internes de l'enveloppe rigide, et
- une étape dans laquelle la porte s'accouple au module de mesure en définissant un deuxième volume de mesure entre ladite face de mesure et la porte en vis-à-vis pour réaliser une mesure de contamination de la porte.

Selon un mode de réalisation, un premier interstice est généré entre l'interface de mesure enveloppe et l'enveloppe rigide accouplée à l'interface de mesure enveloppe. Le jet de gaz injecté dans les buses d'injection est dimensionné par rapport au gaz prélevé pour générer un flux de gaz de fuite à travers le premier interstice, dirigé vers l'extérieur de l'enveloppe rigide. Un deuxième interstice est généré entre l'interface en creux de mesure porte et la porte accouplée à l'interface en creux de mesure porte. Le jet de gaz injecté dans les buses d'injection est dimensionné par rapport au gaz prélevé pour générer un flux de gaz de fuite à travers le deuxième interstice, dirigé vers l'extérieur de l'interface en creux de mesure porte.

En injectant plus de gaz qu'on en prélève, on s'assure de ne pas contaminer ni l'enveloppe rigide, ni la porte.

On obtient ainsi une mesure reproductible, automatique et rapide, pouvant en outre être détaillée quant à la provenance des particules par rapport aux parois internes de l'enceinte de transport.

D'autres avantages et caractéristiques apparaîtront à la lecture de la description d'un exemple illustratif mais non limitatif de la présente invention, ainsi que des dessins annexés sur lesquels :
- la figure 1 représente une vue en perspective d'un premier mode de réalisation d'une station de mesure de la contamination en particules accouplée à une enceinte de transport,
- la figure 2 représente une vue agrandie de l'enveloppe rigide de l'enceinte de transport accouplée à la station de mesure avec le module de mesure en position de mesure,
- la figure 3a représente une vue schématique d'une station de mesure et d'une enceinte de transport,
- la figure 3b représente une vue similaire à la figure 3a, au cours d'une première étape du procédé de mesure dans laquelle le port de chargement de la station de mesure s'accouple à la porte de l'enceinte de transport,
- la figure 3c représente une vue similaire à la figure 3a, au cours d'une deuxième étape du procédé de mesure dans laquelle une porte du port de chargement et la porte accouplée sont déplacées dans la chambre à environnement contrôlé,
- la figure 3d représente une vue similaire à la figure 3a, au cours d'une quatrième étape du procédé de mesure dans laquelle l'interface de mesure enveloppe est accouplée à l'enveloppe rigide,
- la figure 3e représente une vue similaire à la figure 3a, au cours d'une septième étape du procédé de mesure dans laquelle la porte est accouplée au module de mesure,
- la figure 4 montre un exemple de réalisation d'une tête de mesure,
- la figure 5 représente une vue de côté de la porte prise en sandwich entre une interface en creux de mesure porte et la porte du port de chargement,
- la figure 6 représente une vue en perspective d'un deuxième mode de réalisation d'une station de mesure de la contamination en particules, et
- la figure 7 représente une vue schématique de la station de mesure en particules de la figure 6.

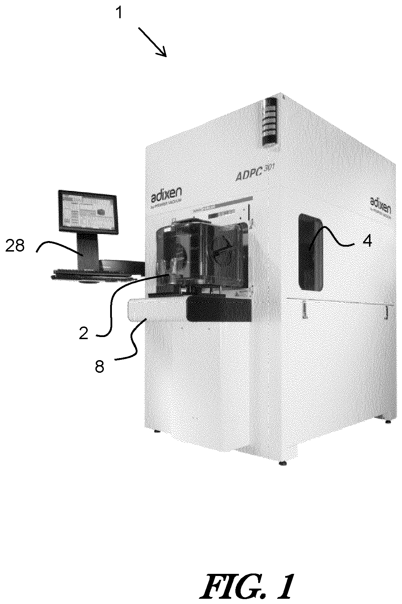

On a représenté sur la figure 1, une station de mesure 1 de la contamination en particules accouplée à une enceinte de transport pour le convoyage et le stockage atmosphérique de substrats semi-conducteurs de type FOUP.

Bien que les figures illustrent une station de mesure susceptible de s'accoupler à une enceinte de transport de type FOUP, la station de mesure peut être adaptée pour d'autres types d'enceintes de transport pour le convoyage et le stockage atmosphérique de substrats semi-conducteurs telles que notamment celles standardisées de type SMIF, FOSB, RSP ou MRP.

Ces enceintes de transport présentent une atmosphère intérieure confinée sous pression atmosphérique d'air ou d'azote, c'est-à-dire à une pression sensiblement équivalente à celle de l'environnement d'utilisation de la salle blanche, mais séparée de celle-ci.

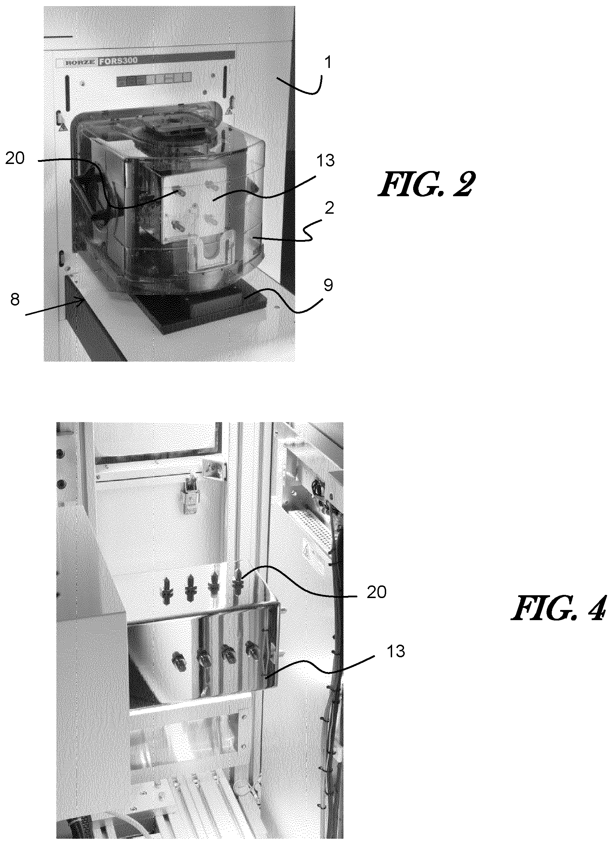

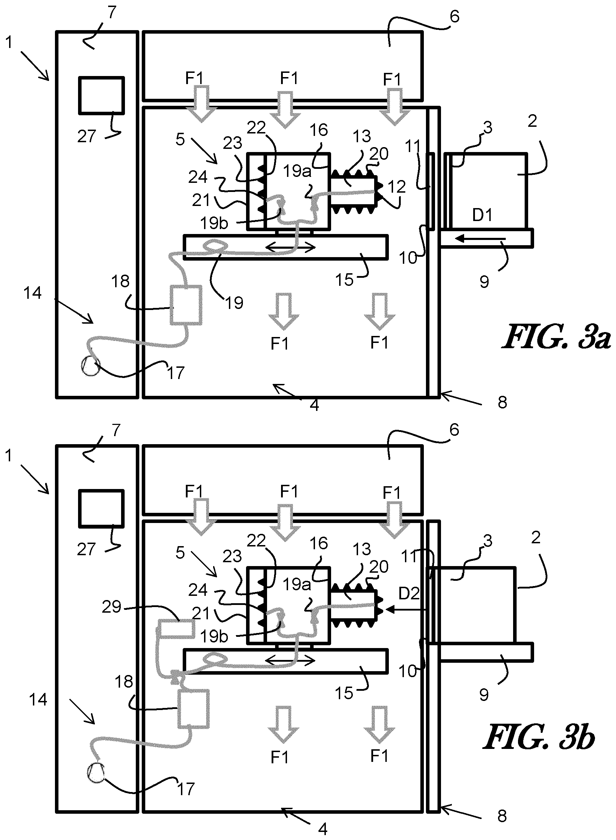

Comme on peut le voir sur les figures 2 et 3a, les enceintes de transport comportent une enveloppe rigide périphérique 2 de forme générale sensiblement parallélépipédique munie d'une ouverture pouvant être obturée par une porte 3 amovible dimensionnée pour permettre l'introduction et l'extraction de substrats. L'enveloppe 2 et la porte 3 sont formées de matériaux tels que le polycarbonate. Dans le cas des enceintes de transport de type FOUP, l'enveloppe rigide 2 présente une paroi de fond sensiblement cylindrique. Les parois internes latérales, de fond et la porte 3 sont munies de fentes (ou « slot » en anglais) pour soutenir les substrats. L'enceinte est relativement étanche, mais le niveau d'étanchéité est tel que de légères fuites peuvent se produire à travers un joint d'étanchéité agencé entre l'enveloppe rigide 2 et la porte 3. Certaines enceintes de transport, notamment les enceintes de type FOUP, comportent des passages de gaz filtrés pour équilibrer la pression entre l'intérieur et l'extérieur de l'enceinte de transport.

Pour les mesures, les enceintes de transport sont vidées de leurs substrats.

Comme visible sur la représentation schématique de la figure 3a, la station de mesure 1 comporte une chambre à environnement contrôlé 4 et un module de mesure 5.

L'atmosphère interne de la chambre à environnement contrôlé 4 est sous pression atmosphérique telle que définie précédemment. La chambre 4 est de type salle blanche. Elle est par exemple certifiée Iso 3, conformément à la norme ISO 146644-1, dite de « mini-environnement ». Pour cela, la chambre à environnement contrôlé 4 peut comporter une unité de filtrage à flux laminaire 6.

L'unité de filtrage à flux laminaire 6 comporte des filtres à air pour filtrer les particules en provenance de l'air extérieur, qui pénètrent dans la chambre à environnement contrôlé 4. L'unité de filtrage à flux laminaire 6 comporte également des moyens diffuseurs de flux pour diffuser l'air filtré en un flux laminaire, par exemple depuis le haut de la station 1 vers le bas comme schématisé par les flèches F1 sur la figure 3a. En outre, le fond de la chambre à environnement contrôlé 4 est perforé pour permettre le balayage par le flux laminaire. L'unité de filtrage à flux laminaire 6 permet ainsi de placer l'atmosphère interne de la chambre à environnement contrôlé 4 sous flux laminaire d'air filtré pour limiter l'introduction d'éventuelles particules générées par la circulation de l'air ou par les composants en mouvements dans la chambre à environnement contrôlé 4 et pour maîtriser leur évacuation.

La station de mesure 1 comporte une armoire électrique 7 permettant l'alimentation et le logement de toute ou partie des composants électriques de la station. L'armoire électrique 7 est avantageusement déportée latéralement de la chambre à environnement contrôlé 4, de manière à être à l'écart du flux laminaire d'air filtré, évitant ainsi la contamination de la chambre à environnement contrôlé 4 par les différents composants logés dans l'armoire électrique 7.

La chambre à environnement contrôlé 4 comporte un accès 10 latéral et un port de chargement 8 (« ou load port » en anglais) agencé sous l'accès 10.

Le port de chargement 8 est susceptible de s'accoupler d'une part à l'enveloppe rigide 2 et d'autre part à la porte 3 de l'enceinte de transport pour déplacer la porte 3 dans la chambre à environnement contrôlé 4 et mettre l'intérieur de l'enveloppe rigide 2 en communication avec l'intérieur de la chambre à environnement contrôlé 4.

Le port de chargement 8 comporte un plateau 9 permettant la réception et le positionnement d'une enceinte de transport. Le plateau 9 peut comporter un capteur de présence adapté pour contrôler que le modèle d'enceinte de transport est bien celui qui est compatible avec la station de mesure 1 réceptionnant l'enceinte. En outre, pour s'accoupler à l'enveloppe rigide 2, le plateau 9 du port de chargement 8 comporte un moyen d'arrimage pour d'une part, clamper l'enveloppe rigide 2 et d'autre part, l'avancer contre l'accès 10 de la chambre à environnement contrôlé 4 (flèche D1 sur la figure 3a).

Le port de chargement 8 comporte également une porte de port de chargement 11. La porte de port de chargement 11 présente les sensiblement les mêmes dimensions que la porte 3 de l'enceinte de transport. La porte de port de chargement 11 permet notamment de fermer l'accès 10 de la chambre à environnement contrôlé 4 en l'absence d'enceinte de transport. Elle comporte en outre des moyens d'actionnement de verrou pour verrouiller et déverrouiller des organes de verrouillage de la porte 3.

Les organes de verrouillage de la porte 3, connus en soi, comprennent par exemple des loquets, portés par la porte 3, actionnés en coulissement radial ou latéral et s'engageant dans l'enveloppe rigide 2 de l'enceinte de transport lorsque l'enceinte de transport est fermée.

Une fois les organes de verrouillage déverrouillés, les moyens d'actionnement de verrou solidarisent de manière réversible la porte 3 à la porte de port de chargement 11. L'ensemble des portes 3,11 peut alors être déplacé d'un seul bloc dans la chambre à environnement contrôlé 4. Pour cela, la porte de port de chargement 11 comporte un mécanisme d'actionnement de porte.

Le mécanisme d'actionnement de porte comporte par exemple un premier axe linéaire motorisé (non représenté) permettant une translation linéaire, telle qu'horizontale, comme représentée par la flèche D2 sur la figure 3b. Ces actionneurs sont avantageusement à paliers électromagnétiques pour être déplacés sans frottements, donc proprement. Le mécanisme d'actionnement de porte est agencé dans la chambre à environnement contrôlé 4 en flux laminaire filtré pour que les éventuelles particules générées par l'actionneur soient évacuées.

L'ensemble des portes 3, 11 est déporté hors de la zone frontale de l'accès 10, par exemple à proximité de la paroi interne de la chambre à environnement contrôlé 4 opposée à l'accès 10. Selon un autre exemple non représenté, le mécanisme d'actionnement de porte déplace l'ensemble des portes 3, 11 vers le bas de la chambre à environnement contrôlé 4 plutôt qu'horizontalement.

Lorsque la porte 3 est déplacée à l'écart de l'enveloppe rigide 2, le volume intérieur de l'enveloppe rigide 2 est en communication avec le volume intérieur de la chambre à environnement contrôlé 4.

Le module de mesure 5 comporte une interface de mesure enveloppe 16 configurée pour s'accoupler à une enveloppe rigide 2 d'enceinte de transport accouplée à la chambre à environnement contrôlé 4 à la place de la porte 2, une unité de mesure des particules 14 et un mécanisme de déplacement module 15.

L'interface de mesure enveloppe 16 est munie d'au moins deux buses d'injection 20 configurées pour diriger un jet de gaz sur au moins deux endroits distincts de l'enveloppe rigide 2 accouplée, les orientations respectives des buses d'injection 20 étant fixes par rapport à l'enveloppe rigide 2 accouplée. L'interface de mesure enveloppe 16 comporte également un premier orifice de prélèvement 12 raccordé à l'unité de mesure des particules 14.

L'orifice de prélèvement 12 et les buses d'injection 20 sont par exemple agencés sur une tête de mesure 13 faisant saillie d'une base de l'interface de mesure enveloppe 16.

Les buses d'injection 20 comportent des injecteurs en matériau dur, tel qu'en rubis, en saphir ou Zircone. Les injecteurs sont des cylindres creux en matériau dur dont le diamètre intérieur peut être défini avec une très haute précision (de l'ordre de quelques µm), ce diamètre interne définissant le couple de débit d'injection du gaz et de variation de pression entre la pression atmosphérique de la chambre à environnement contrôlé 4 et la pression d'arrivée des gaz. Les injecteurs en matériau dur sont inusables, ce qui évite les dérives de dimension dans le temps et assure qu'ils puissent être réalisés très précisément, ce qui permet une bonne reproductibilité des mesures.

Les injecteurs sont raccordés à des systèmes d'amenée de gaz pourvus de vannes d'isolation (non représentées), passant dans un logement 5a du module de mesure 5. Le gaz injecté est un gaz neutre tel que de l'azote. Les buses d'injection 20 sont en outre pourvues de filtres à particules pour filtrer d'éventuelles particules polluantes du gaz injecté.

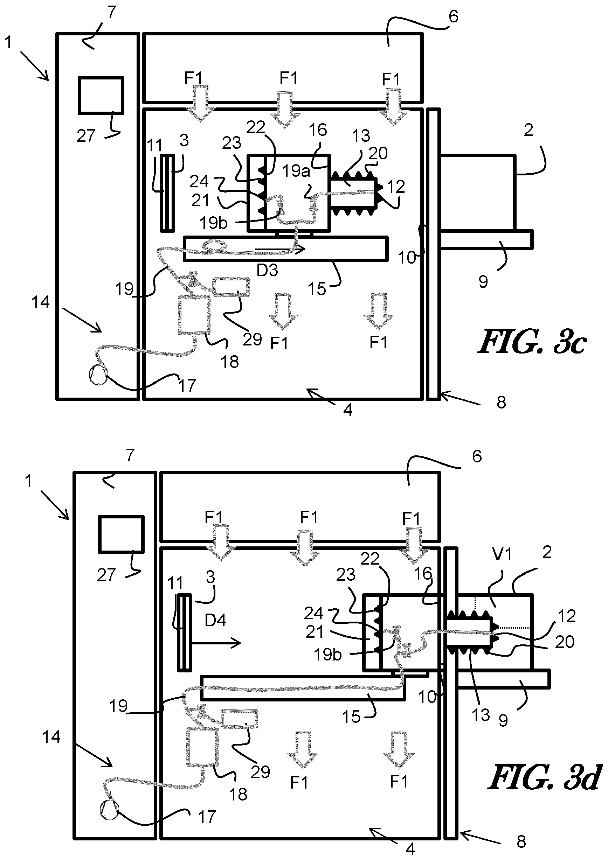

Les buses d'injection 20 sont par exemple configurées pour diriger un jet de gaz dans au moins deux directions perpendiculaires entre elles et perpendiculaires aux parois de l'enveloppe rigide 2 accouplée à la chambre à environnement contrôlé 4 (voir les exemples de jet de gaz représentés en pointillés sur la figure 3d). Le jet de gaz confiné perpendiculairement à la paroi permet d'améliorer l'impact sur les parois pour un décrochage efficace des particules.

La tête de mesure 13 présente par exemple une forme générale parallélépipédique sensiblement complémentairement à la forme interne de l'enveloppe rigide 2. Chacune des cinq faces de la tête de mesure 13 faisant saillie de la base de l'interface de mesure enveloppe 16, comporte au moins une buse d'injection 20 de manière à diriger un jet de gaz dans une direction sensiblement orthogonale à la face de la tête de mesure 13. On peut ainsi mesurer individuellement chacune des cinq faces de l'enveloppe rigide 2.

En outre, chaque face peut comporter plusieurs buses d'injection 20 par exemple configurées pour diriger des jets de gaz dans des directions parallèles entre elles.

Par exemple et comme représenté sur la figure 2, la tête de mesure 13 comporte quatre buses d'injection 20 sur chacune des cinq faces en saillie. Les quatre buses d'injection 20 d'une face s'inscrivent aux quatre coins d'une forme carrée.

Selon un autre exemple représenté sur la figure 4, les quatre buses d'injection 20 de chaque face sont alignées suivant une ligne sensiblement médiane et horizontale.

Pour augmenter la fiabilité de la mesure, on peut par exemple localiser les jets de gaz dans des zones particulièrement critiques de l'enveloppe rigide 2, tel qu'au niveau des fentes de support des substrats ou dans les coins de l'enveloppe rigide 2. On peut aussi couvrir un maximum de la surface de la paroi interne, notamment en augmentant le nombre de buses d'injection 20 par face.

Le premier orifice de prélèvement 12 est par exemple ménagé sur une des faces de la tête de mesure 13.

La tête de mesure 13 est configurée pour que la distance entre la sortie d'une buse d'injection 20 et la paroi interne d'une enveloppe rigide 2 accouplée soit inférieure à quelques centimètres, telle que comprise entre 1 mm et 10 cm. Pour optimiser l'arrachage des particules, le débit des buses d'injection 20 est par exemple compris entre 10 et 30 l/min, tel que de l'ordre de 20 l/min, suivant le nombre de buses d'injection 20, le débit diminuant avec l'augmentation du nombre de buses d'injection 20. La variation de pression entre la pression atmosphérique de la chambre à environnement contrôlé 4 et la pression d'arrivée des gaz est par exemple de l'ordre de 3 à 4 bars.

Le mécanisme de déplacement module 15 est configuré pour déplacer l'interface de mesure enveloppe 16 dans entre une position de repos (figure 3a) et une position de mesure l'enveloppe rigide 2 accouplée à la chambre à environnement contrôlé 4 (figure 3d). Le mécanisme de déplacement module 15 comporte un deuxième axe linéaire motorisé permettant par exemple deux translations linéaires, telle qu'horizontale comme représentée par la flèche D3 sur la figure 3c, et verticale pour décaler la tête de mesure 13 hors d'une zone frontale de l'accès 10 pendant le déplacement de la porte 3 vers/à l'écart de l'enveloppe rigide 2. Comme pour le mécanisme d'actionnement de porte, l'actionneur du mécanisme de déplacement module 15 est avantageusement à paliers électromagnétiques et est placé dans la chambre à environnement contrôlé 4 sous flux laminaire d'air filtré.

La tête de mesure 13 fait saillie de la base de l'interface de mesure enveloppe 16 en direction de l'accès 10 de la chambre à environnement contrôlé 4. La base de l'interface de mesure enveloppe 16 présente sensiblement la même forme et les mêmes dimensions qu'une porte 3 d'enceinte de transport, pour être facilement accouplée à une enveloppe rigide 2 d'enceinte de transport à la place de la porte 3. La tête de mesure 13 est par exemple solidarisée au centre de la base de l'interface de mesure enveloppe 16.

Lorsque l'interface de mesure enveloppe 16 est déplacée dans l'enveloppe rigide 2 accouplée à la chambre à environnement contrôlé 4 en position de mesure, l'interface de mesure enveloppe 16 ferme l'enveloppe rigide 2 à la place de la porte 3 en définissant un premier volume de mesure V1 (figure 3d). La tête de mesure 13 est reçue dans ce premier volume de mesure V1, ce qui place l'intérieur de l'enveloppe rigide 2 en communication avec le premier orifice de prélèvement 12 raccordé à l'unité de mesure des particules 14, ainsi qu'avec les buses d'injection 20. On profite ainsi de la forme en coquille de l'enveloppe périphérique de l'enceinte de transport pour définir un premier volume de mesure V1 entre l'enveloppe rigide 2 et l'interface de mesure enveloppe 16. La mesure est alors réalisée dans ce premier volume de mesure V1 confiné, séparé du reste de la station de mesure 1.

L'interface de mesure enveloppe 16 ne referme pas l'enveloppe rigide 2 de manière étanche, mais ménage un faible premier interstice entre les deux pour le passage d'un flux de fuite. Le flux d'injection de gaz injecté dans les buses d'injection 20 est dimensionné pour placer le premier volume de mesure V1 en légère surpression par rapport à l'environnement extérieur, favorisant ainsi le sens du flux de gaz vers l'extérieur à travers le premier interstice, ce qui réduit les risques de contamination en particules.

L'unité de mesure des particules 14 comporte par exemple une pompe à vide 17, un compteur de particules 18 raccordé en amont de la pompe à vide 17 et une ligne de prélèvement 19 en amont du compteur de particules 18 comme illustré en figure 3a. La pompe à vide 17 est par exemple déportée dans l'armoire électrique 7.

La ligne de prélèvement 19 est raccordée à son extrémité au premier orifice de prélèvement 12 de la tête de mesure 13. La ligne de prélèvement 19 peut comporter une première vanne d'isolation 19a agencée entre le premier orifice de prélèvement 12 et le compteur de particules 18. La ligne de prélèvement 19 est suffisamment souple et longue pour permettre les déplacements de la tête de mesure 13. L'unité de mesure des particules 14 peut comporter par exemple une chaîne porte-câble pour porter et guider la ligne de prélèvement 19. Le compteur de particules 18 est par exemple logé dans la chambre à environnement contrôlé 4, au plus près de l'orifice de prélèvement 12 de manière à limiter la longueur de la ligne de prélèvement 19 raccordée au compteur de particules 18.

En outre, l'unité de mesure des particules 14 peut comporter des moyens de nettoyage 29 configurés pour injecter un gaz de purge dans la ligne de prélèvement 19 afin de la purger des particules qui y seraient éventuellement logées.

L'échantillon de gaz est prélevé dans le premier volume de mesure V1 de l'enveloppe rigide 2 accouplée à l'interface de mesure enveloppe 16 par aspiration par le premier orifice de prélèvement 12 de la tête de mesure 13. La quantité de particules contenues dans l'échantillon de gaz prélevé est déterminée par le compteur de particules 18. Le compteur de particules 18 est par exemple de type aérosol, c'est-à-dire qu'il permet de donner une information quantitative des particules en suspension dans un environnement gazeux. Il est par exemple basé sur la technologie laser. Le flux de pompage de la pompe à vide 17 est par exemple de l'ordre de 1,7 m³/h.

Les moyens de positionnement, les moyens de contrôle du modèle d'enceinte de transport, les moyens d'actionnement de verrou, les mécanismes d'actionnement de porte du port de chargement et les moyens d'injection de gaz sont pilotés par une unité de traitement 27 de la station de mesure 1. L'unité de traitement 27 est en outre configurée pour commander l'injection sélective du gaz dans les buses d'injection 20. L'unité de traitement 27 est raccordée à une interface utilisateur 28, comprenant par exemple notamment un écran et un clavier visibles sur la figure 1.

La station de mesure 1 est également adaptée pour mesurer la porte 3 de l'enceinte de transport.

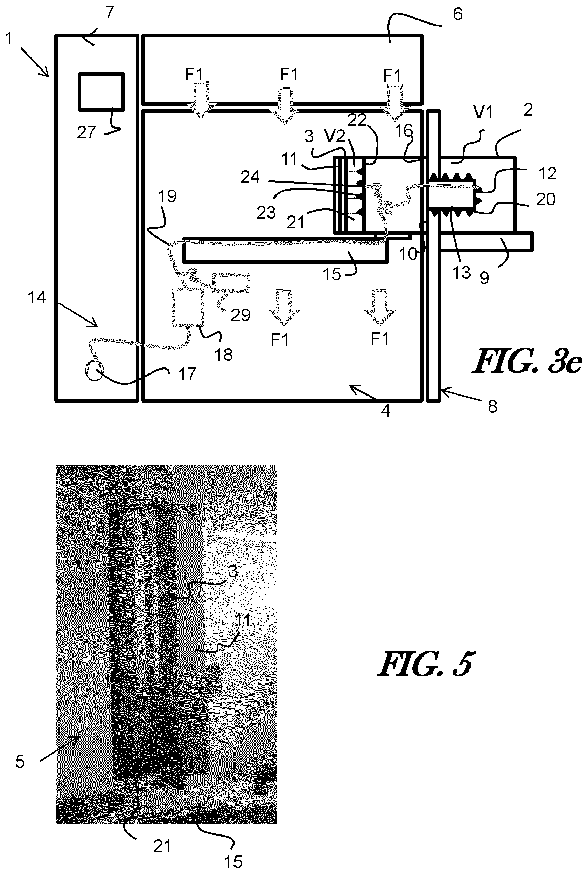

Pour cela, le module de mesure 5 comporte une interface en creux de mesure porte 21 configurée pour s'accoupler à la porte 3 en définissant un deuxième volume de mesure V2 entre une face de mesure 22 du module de mesure 5 et la porte 3 en vis-à-vis (figure 3e et 5). Le mécanisme d'actionnement de porte du port de chargement 8 est par exemple susceptible de déplacer la porte 3 en direction de l'interface en creux de mesure porte 21 après que la tête de mesure 13 ait été déplacée dans l'enveloppe rigide 2.

La face de mesure 22 présente au moins une buse d'injection 23 et un deuxième orifice de prélèvement 24. Par exemple, la face de mesure 22 comporte quatre buses d'injections 23. L'interface en creux de mesure porte 21 met alors en communication l'intérieur du deuxième volume de mesure V2 avec le deuxième orifice de prélèvement 24 raccordé à l'unité de mesure des particules 14 et avec les buses d'injections 23.

Etant donné que la porte 3 ne permet pas de prédéfinir une portion volumique permettant de confiner les particules décrochées à mesurer, c'est l'interface en creux de mesure porte 21 qui est adaptée pour définir un deuxième volume de mesure V2 dans lequel les buses d'injections 23 soufflent pour décrocher les particules de la porte 3 et dans lequel un deuxième orifice de prélèvement 24 raccordé à l'unité de mesure des particules 14 prélève l'échantillon gazeux au moment de la réalisation d'une mesure (figure 3e).

L'interface en creux de mesure porte 21 présente par exemple une forme générale de cadre dont les dimensions périphériques sont sensiblement équivalentes à celles d'une porte 3 et dont l'épaisseur est sensiblement équivalente à la distance optimale entre la sortie d'une buse d'injection 20 et la paroi interne d'une enveloppe rigide 2 accouplée. La distance optimale est de l'ordre de quelques centimètres telle que comprise entre 1 et 10 cm. Le deuxième volume de mesure V2 représente ainsi un volume d'environ un cinquième du volume interne de l'enceinte de transport.

Les buses d'injections 23 sont similaires aux buses d'injection 20 de la tête de mesure 13 et sont par exemple configurées pour diriger un jet de gaz dans une direction sensiblement orthogonale à la face de mesure 22 (voir les exemples de jet de gaz représentés en pointillés sur la figure 3e).

L'interface en creux de mesure porte 21 ne se referme pas de manière étanche sur la porte 3, mais ménage un faible deuxième interstice entre les deux pour le passage d'un flux de fuite. Le flux d'injection de gaz injecté dans les buses d'injection 20 est dimensionné pour placer le deuxième volume de mesure V2 en légère surpression par rapport à l'environnement extérieur, favorisant ainsi le sens du flux de gaz vers l'extérieur à travers le deuxième interstice, ce qui réduit les risques de contamination en particules.

L'interface de mesure enveloppe 16 et l'interface en creux de mesure porte 21 sont par exemple agencées dos à dos. La tête de mesure 13 est susceptible d'être translatée dans l'enveloppe rigide 2 accouplée et la porte 3 est susceptible d'être translatée en direction de l'interface en creux de mesure porte 21.

La ligne de prélèvement 19 comporte un dispositif à vanne pour basculer sélectivement vers le premier ou le deuxième orifice de prélèvement 12, 24 (figure 3a).

Le dispositif à vanne comporte par exemple une première vanne d'isolation 19a agencée entre le premier orifice de prélèvement 12 et le compteur de particules 18 et une deuxième vanne d'isolation 19b agencée entre le deuxième orifice de prélèvement 24 et le compteur de particules 18. Les première et deuxième vannes d'isolation 19a, 19b peuvent être pilotées par l'unité de traitement 27 pour mesurer sélectivement la porte 3 de l'enceinte de transport ou une des parois internes de l'enveloppe rigide 2. On utilise ainsi un seul compteur de particules 18 pour mesurer à la fois la porte 3 et les parois internes de l'enveloppe rigide 2. Selon un autre exemple le dispositif à vanne comporte une vanne trois voies.

En position de repos, le module de mesure 5 est agencé dans la chambre à environnement contrôlé 4 dont l'accès 10 est fermée par la porte de port de chargement 11 (figure 3a).

Puis, lorsqu'un opérateur ou un robot place une enceinte de transport sur le plateau 9 du port de chargement 8, le port de chargement 8 positionne et contrôle le modèle d'enceinte de transport, puis clampe l'enveloppe rigide 2 de l'enceinte et l'avance contre l'accès 10 de la chambre à environnement contrôlé 4 (flèche D1 sur la figure 3a).

Les moyens d'actionnement de verrou de la porte de port de chargement 11 déverrouillent alors les organes de verrouillage de la porte 3 et solidarisent la porte 3 à la porte de port de chargement 11 (première étape, figure 3b).

L'ensemble des portes 3-11 est alors déplacé dans la chambre à environnement contrôlé 4 à l'écart de l'accès 10 (flèche D1 sur la figure 3b) mettant le volume intérieur de l'enveloppe rigide 2 en communication avec le volume intérieur de la chambre à environnement contrôlé 4 (deuxième étape, figure 3c). La station de mesure 1 peut comporter un capteur de sécurité permettant de vérifier que l'enveloppe rigide 2 est bien vidée de ses substrats après ouverture de l'enceinte de transport.

Dans une troisième étape, l'interface de mesure enveloppe 16 est déplacée en direction de l'enveloppe rigide 2.

Dans une quatrième étape, l'interface de mesure enveloppe 16 s'accouple à l'enveloppe rigide 2 à la place de la porte 3 en position de mesure. A l'état accouplé, la tête de mesure 13 est immobilisée dans le premier volume de mesure V1 définit par l'interface de mesure enveloppe 16 et l'enveloppe rigide 2 accouplée. Ce premier volume de mesure V1 est donc en communication d'une part, avec le premier orifice de prélèvement 12 ménagé dans la tête de mesure 13 et raccordé à l'unité de mesure des particules 14 et d'autre part, avec les buses d'injection 20 de la tête de mesure 13 (figure 3d).

Dans une cinquième étape, un jet de gaz est injecté simultanément dans les buses d'injection 20 de même orientation. Par exemple, un jet de gaz est injecté dans les quatre buses d'injection 20 d'une même face de la tête de mesure 13. Le jet de gaz décroche un échantillon de particules présentes sur les parois internes de l'enveloppe rigide 2 accouplée. La distance entre les sorties des buses d'injection 20 et les parois interne est maîtrisée (du fait notamment des dimensions de la tête de mesure 13), ce qui permet de décrocher les particules de façon reproductible d'une enceinte de transport à l'autre.

Le gaz est prélevé dans le premier volume de mesure V1 par aspiration par la ligne de prélèvement 19. La quantité de particules contenues dans l'échantillon de gaz prélevé est déterminée en continu par le compteur de particules 18.

Le flux d'injection de gaz place le premier volume de mesure V1 en légère surpression par rapport à l'environnement extérieur, favorisant ainsi le sens du flux de gaz vers l'extérieur à travers le premier interstice entre l'interface de mesure enveloppe 16 et l'enveloppe rigide 2, ce qui réduit les risques de contamination en particules de l'enveloppe rigide 2.

Puis l'opération de mesure est réitérée pour les buses d'injection 20 de même orientation de chaque face de la tête de mesure 13.

L'unité de traitement 27 informe l'utilisateur de l'état de propreté face par face de l'enceinte de transport.

Une fois que l'interface de mesure enveloppe 16 est accouplée à l'enveloppe rigide 2, la porte de port de chargement 11 et la porte 3 accouplée sont déplacées en direction de l'interface en creux de mesure porte 21 par l'action du mécanisme d'actionnement de porte (sixième étape, flèche D4 sur la figure 3d).

Puis dans une septième étape, la porte 3 s'accouple au module de mesure 5 en définissant un deuxième volume de mesure V2 entre une face de mesure du module de mesure 5 et la porte 3 en vis-à-vis (figure 3e). A l'état accouplé, l'interface en creux de mesure porte 21 est immobilisée contre la face de mesure 22. Le deuxième volume de mesure V2 est donc en communication d'une part, avec le deuxième orifice de prélèvement 24 ménagé dans la face de mesure 22 et raccordé à l'unité de mesure des particules 14 et d'autre part, avec les buses d'injections 23 de la face de mesure 22. La septième étape peut être réalisée successivement à la quatrième étape.

Dans une huitième étape, les buses d'injections 23 injectent un jet de gaz en direction de la porte 3. Le jet de gaz décroche un échantillon de particules présentes sur la porte 3. La distance entre les sorties des buses d'injection 23 et la porte 3 est maîtrisée, ce qui permet de décrocher les particules de façon reproductible d'une enceinte de transport à l'autre.

Le gaz est prélevé dans le deuxième volume de mesure V2 par aspiration par la ligne de prélèvement 19 après fermeture de la première vanne d'isolation 19a et ouverture de la deuxième vanne d'isolation 19b. La quantité de particules contenues dans l'échantillon de gaz prélevé est déterminée en continu par le compteur de particules 18. Le flux d'injection de gaz place le deuxième volume de mesure V2 en légère surpression par rapport à l'environnement extérieur, favorisant ainsi le sens du flux de gaz vers l'extérieur à travers le deuxième interstice entre l'interface en creux de mesure porte 21 et la porte 3, ce qui réduit les risques de contamination en particules.

L'unité de traitement 27 informe l'utilisateur de l'état de propreté face par face de l'enceinte de transport, y compris la porte 3.

Lorsque les mesures sont terminées, l'ensemble de portes 3, 11 est déplacé à l'écart de l'interface en creux de mesure porte 21, la tête de mesure 13 est retirée de l'enveloppe rigide 2 et l'enceinte de transport est refermée soit pour être envoyée au nettoyage, soit pour poursuivre l'opération de transport et de de convoyage, selon son état de propreté.

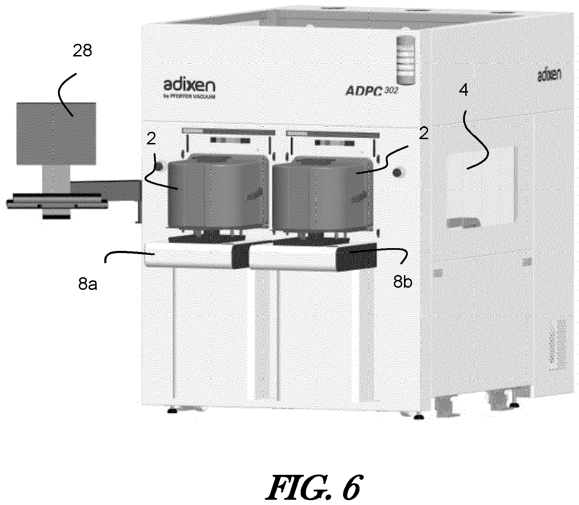

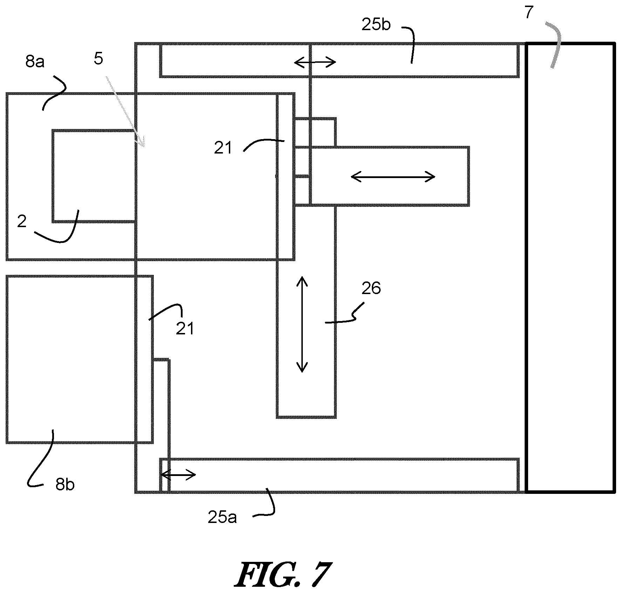

Selon un deuxième mode de réalisation de la station de mesure représentée sur les figures 6 et 7, la chambre à environnement contrôlé 4 comporte deux ports de chargement 8a, 8b susceptibles de s'accoupler à une enceinte de transport respective.

Chaque port de chargement 8a, 8b comporte son propre mécanisme d'actionnement de porte 25a, 25b.

Le mécanisme de déplacement module 26 est configuré pour déplacer la tête de mesure 13 en position de mesure dans l'une ou l'autre des enveloppe rigides 2 accouplées. Pour cela, le mécanisme de déplacement module 26 permet par exemple à la tête de mesure 13 d'être déportée latéralement à son déplacement axial vers la chambre à environnement contrôlé 4. La tête de mesure 13 peut ainsi être déportée de l'accès 10 de la chambre à environnement contrôlé 4 pour laisser libre champ au mécanisme d'actionnement de porte 25a ou 25b.

## Revendications

1. Station de mesure de la contamination en particules d'une enceinte de transport pour le convoyage et le stockage atmosphérique de substrats semi-conducteurs, ladite enceinte de transport comprenant une enveloppe rigide (2) munie d'une ouverture et une porte (3) amovible permettant d'obturer l'ouverture, la station de mesure comportant :
- une chambre à environnement contrôlé (4) comportant au moins un port de chargement (8) susceptible de s'accoupler d'une part à l'enveloppe rigide (2) et d'autre part à la porte (3) de l'enceinte de transport, pour déplacer la porte (3) dans la chambre à environnement contrôlé (4),
- un module de mesure (5) comportant une unité de mesure des particules (14), ainsi qu'une interface de mesure enveloppe (16) configurée pour s'accoupler à l'enveloppe rigide (2) accouplée à la chambre à environnement contrôlé (4) à la place de la porte (3), en définissant un premier volume de mesure (V1), ladite interface de mesure enveloppe (16) comportant au moins une buse d'injection (20) et un premier orifice de prélèvement (12) raccordé à l'unité de mesure des particules (14),
**caractérisé en ce que** ledit module de mesure (5) comporte une interface en creux de mesure porte (21) configurée pour s'accoupler à la porte (3) en définissant un deuxième volume de mesure (V2) entre une face de mesure (22) du module de mesure (5) et la porte (3) en vis-à-vis, ladite face de mesure (22) présentant au moins une buse d'injection (23) et un deuxième orifice de prélèvement (24) raccordé à l'unité de mesure des particules (14).

2. Station de mesure selon la revendication 1, **caractérisée en ce que** l'interface en creux de mesure porte (21) présente une forme générale de cadre.

3. Station de mesure selon l'une des revendications 1 ou 2, **caractérisée en ce que** la porte (3) est susceptible d'être déplacée en direction de l'interface en creux de mesure porte (21).

4. Station de mesure selon l'une des revendications 1 à 3, **caractérisée en ce que** le module de mesure (5) comporte un mécanisme de déplacement module (15 ; 26) configuré pour déplacer l'interface de mesure enveloppe (16) entre une position de repos et une position de mesure dans l'enveloppe rigide (2) accouplée.

5. Station de mesure selon les revendications 3 et 4, **caractérisée en ce que** l'interface de mesure enveloppe (16) et l'interface en creux de mesure porte (21) sont agencées dos à dos, **en ce que** l'interface de mesure enveloppe (16) est susceptible d'être translatée dans l'enveloppe rigide (2) accouplée et **en ce que** la porte (3) est susceptible d'être translatée en direction de l'interface en creux de mesure porte (21).

6. Station de mesure selon l'une des revendications précédentes, **caractérisée en ce que** la ligne de prélèvement (19) de l'unité de mesure des particules (14) comporte un dispositif à vanne (29a, 29b) pour basculer sélectivement vers le premier ou le deuxième orifice de prélèvement (12, 24).

7. Station de mesure selon l'une des revendications précédentes, **caractérisée en ce que** l'interface de mesure enveloppe (16) comporte au moins deux buses d'injection (20) configurées pour diriger un jet de gaz sur au moins deux endroits distincts de l'enveloppe rigide (2) accouplée à la chambre à environnement contrôlé (4), les orientations respectives des buses d'injection (20) étant fixes par rapport à l'enveloppe rigide (2) accouplée.

8. Station de mesure selon l'une des revendications précédentes, **caractérisée en ce que** l'interface de mesure enveloppe (16) comporte une tête de mesure (13) faisant saillie d'une base de l'interface de mesure enveloppe (16).

9. Station de mesure selon la revendication 8, **caractérisée en ce que** ladite tête de mesure (13) présente une forme générale parallélépipédique et **en ce que** chacune des cinq faces de la tête de mesure (13) faisant saillie de la base de l'interface de mesure enveloppe (16) comporte au moins une buse d'injection (20).

10. Station de mesure selon l'une des revendications 7 ou 9, **caractérisée en ce qu'**elle comporte une unité de traitement (27) configurée pour commander l'injection sélective du gaz dans les buses d'injection (20, 23).

11. Procédé de mesure de la contamination en particules d'une enceinte de transport pour le convoyage et le stockage atmosphérique de substrats semi-conducteurs, mis en oeuvre dans une station de mesure selon l'une des revendications 1 à 10, **caractérisé en ce qu'**il comporte :
- une étape dans laquelle le module de mesure (5) s'accouple à l'enveloppe rigide (2) en définissant un premier volume de mesure (V1) entre l'interface de mesure enveloppe (16) et l'enveloppe rigide (2) accouplée pour réaliser une mesure de contamination des parois internes de l'enveloppe rigide (2), et
- une étape dans laquelle la porte (3) s'accouple au module de mesure (5) en définissant un deuxième volume de mesure (V2) entre ladite face de mesure (22) et la porte (3) en vis-à-vis pour réaliser une mesure de contamination de la porte (3).

12. Procédé de mesure selon la revendication 11, **caractérisé en ce qu'**un premier interstice est généré entre l'interface de mesure enveloppe (16) et l'enveloppe rigide (2) accouplée à l'interface de mesure enveloppe (16), le jet de gaz injecté dans les buses d'injection (20) étant dimensionné pour générer un flux de gaz de fuite à travers le premier interstice, dirigé vers l'extérieur de l'enveloppe rigide (2) et **en ce qu'**un deuxième interstice est généré entre l'interface en creux de mesure porte (21) et la porte (3) accouplée à l'interface en creux de mesure porte (21), le jet de gaz injecté dans les buses d'injection (23) étant dimensionné pour générer un flux de gaz de fuite à travers le deuxième interstice, dirigé vers l'extérieur de l'interface en creux de mesure porte (21).

## Patentansprüche

1. Station zur Messung der Partikelkontamination eines Transportbehälters zur Beförderung und atmosphärischen Lagerung von Halbleitersubstraten, wobei der Transportbehälter eine mit einer Öffnung versehene steife Hülle (2) und eine entfernbare Tür (3) enthält, die es ermöglicht, die Öffnung zu verschließen, wobei die Messstation aufweist:
- eine Reinraumkammer (4), die mindestens einen Beladeport (8) aufweist, der einerseits mit der steifen Hülle (2) und andererseits mit der Tür (3) des Transportbehälters gekoppelt werden kann, um die Tür (3) in die Reinraumkammer (4) zu verschieben,
- ein Messmodul (5), das eine Messeinheit der Partikel (14), sowie eine Hüllen-Messschnittstelle (16) aufweist, die konfiguriert ist, mit der mit der Reinraumkammer (4) gekoppelten steifen Hülle (2) anstelle der Tür (3) gekoppelt zu werden, indem ein erstes Messvolumen (V1) definiert wird, wobei die Hüllen-Messschnittstelle (16) mindestens eine Einspritzdüse (20) und eine erste Entnahmeöffnung (12) aufweist, die mit der Partikelmesseinheit (14) verbunden ist,
**dadurch gekennzeichnet, dass** das Messmodul (5) eine vertiefte Tür-Messschnittstelle (21) aufweist, die konfiguriert ist, mit der Tür (3) gekoppelt zu werden, indem ein zweites Messvolumen (V2) zwischen einer Messseite (22) des Messmoduls (5) und der gegenüberliegenden Tür (3) definiert wird, wobei die Messseite (22) mindestens eine Einspritzdüse (23) und eine zweite Entnahmeöffnung (24) aufweist, die mit der Messeinheit der Partikel (14) verbunden ist.

2. Messstation nach Anspruch 1, **dadurch gekennzeichnet, dass** die vertiefte Tür-Messschnittstelle (21) eine allgemeine Rahmenform aufweist.

3. Messstation nach einem der Ansprüche 1 oder 2, **dadurch gekennzeichnet, dass** die Tür (3) in Richtung der vertieften Tür-Messschnittstelle (21) verschoben werden kann.

4. Messstation nach einem der Ansprüche 1 bis 3, **dadurch gekennzeichnet, dass** das Messmodul (5) einen Modul-Verschiebungsmechanismus (15; 26) aufweist, der konfiguriert ist, die Hüllen-Messschnittstelle (16) zwischen einer Ruhestellung und einer Messstellung in der angekoppelten steifen Hülle (2) zu verschieben.

5. Messstation nach den Ansprüchen 3 und 4, **dadurch gekennzeichnet, dass** die Hüllen-Messschnittstelle (16) und die vertiefte Tür-Messschnittstelle (21) Rücken an Rücken angeordnet sind, dass die Hüllen-Messschnittstelle (16) translatorisch in die angekoppelte steife Hülle (2) verschoben werden kann, und dass die Tür (3) translatorisch in Richtung der vertieften Tür-Messschnittstelle (21) verschoben werden kann.

6. Messstation nach einem der vorhergehenden Ansprüche, **dadurch gekennzeichnet, dass** die Entnahmeleitung (19) der Messeinheit der Partikel (14) eine Vorrichtung mit Ventil (29a, 29b) aufweist, um selektiv zur ersten oder zweiten Entnahmeöffnung (12, 24) umzuschalten.

7. Messstation nach einem der vorhergehenden Ansprüche, **dadurch gekennzeichnet, dass** die Hüllen-Messschnittstelle (16) mindestens zwei Einspritzdüsen (20) aufweist, die konfiguriert sind, einen Gasstrahl auf mindestens zwei unterschiedliche Stellen der mit der Reinraumkammer (4) gekoppelten steifen Hülle (2) zu richten, wobei die jeweiligen Ausrichtungen der Einspritzdüsen (20) bezüglich der angekoppelten steifen Hülle (2) ortsfest sind.

8. Messstation nach einem der vorhergehenden Ansprüche, **dadurch gekennzeichnet, dass** die Hüllen-Messschnittstelle (16) einen Messkopf (13) aufweist, der von einer Basis der Hüllen-Messschnittstelle (16) vorsteht.

9. Messstation nach Anspruch 8, **dadurch gekennzeichnet, dass** der Messkopf (13) eine allgemein parallelepipedische Form aufweist, und dass jede der fünf Seiten des Messkopfes (13), der von der Basis der Hüllen-Messschnittstelle (16) vorsteht, mindestens eine Einspritzdüse (20) aufweist.

10. Messstation nach einem der Ansprüche 7 oder 9, **dadurch gekennzeichnet, dass** sie eine Verarbeitungseinheit (27) aufweist, die konfiguriert ist, die selektive Einspritzung des Gases in die Einspritzdüsen (20, 23) zu steuern.

11. Verfahren zur Messung der Partikelkontamination eines Transportbehälters zur Beförderung und atmosphärischen Lagerung von Halbleitersubstraten, angewendet in einer Messstation nach einem der Ansprüche 1 bis 10, **dadurch gekennzeichnet, dass** es aufweist:
- einen Schritt, in dem das Messmodul (5) mit der steifen Hülle (2) gekoppelt wird, indem ein erstes Messvolumen (V1) zwischen der Hüllen-Messschnittstelle (16) und der angekoppelte steifen Hülle (2) definiert wird, um eine Kontaminationsmessung der Innenwände der steifen Hülle (2) durchzuführen, und
- einen Schritt, in dem die Tür (3) mit dem Messmodul (5) gekoppelt wird, indem ein zweites Messvolumen (V2) zwischen der Messseite (22) und der gegenüberliegenden Tür (3) definiert wird, um eine Kontaminationsmessung der Tür (3) durchzuführen.

12. Messverfahren nach Anspruch 11, **dadurch gekennzeichnet, dass** ein erster Zwischenraum zwischen der Hüllen-Messschnittstelle (16) und der mit der Hüllen-Messschnittstelle (16) gekoppelten steifen Hülle (2) erzeugt wird, wobei der in die Einspritzdüsen (20) eingespritzte Gasstrahl bemessen ist, um einen Leckgasstrom durch den ersten Zwischenraum zu erzeugen, der zur Außenseite der steifen Hülle (2) gerichtet ist, und dass ein zweiter Zwischenraum zwischen der vertieften Tür-Messschnittstelle (21) und der mit der vertieften Tür-Messschnittstelle (21) gekoppelten Tür (3) erzeugt wird, wobei der in die Einspritzdüsen (23) eingespritzte Gasstrahl bemessen ist, um einen Leckgasstrom durch den zweiten Zwischenraum zu erzeugen, der zur Außenseite der vertieften Tür-Messschnittstelle (21) gerichtet ist.

## Claims

1. Station for measuring particle contamination of a transport carrier for conveying and storing semiconductor substrates at atmospheric pressure, said transport carrier comprising a rigid casing (2) containing an aperture and a removable door (3) allowing the aperture to be closed, the measuring station comprising:
- a controlled environment chamber (4) comprising at least one load port (8) capable of coupling, on the one hand, to the rigid casing (2), and on the other hand, to the door (3) of the transport carrier, in order to move the door (3) into the controlled environment chamber (4); and
- a measuring module (5) comprising a particle measuring unit (14), and a casing-measuring interface (16) configured to couple to the rigid casing (2) coupled to the controlled environment chamber (4) in the place of the door (3), thereby defining a first measuring volume (V1), said casing-measuring interface (16) comprising at least one injecting nozzle (20) and a first sampling orifice (12) connected to the particle measuring unit (14);
**characterized in that** said measuring module (5) comprises: a hollow, door-measuring interface (21) configured to couple to the door (3), thereby defining a second measuring volume (V2) between a measuring face (22) of the measuring module (5) and the opposite door (3), said measuring face (22) comprising at least one injecting nozzle (23) and a second sampling orifice (24) connected to the particle measuring unit (14).

2. Measuring station according to Claim 1, **characterized in that** the hollow, door-measuring interface (21) has a generally frame-like shape.

3. Measuring station according to either of Claims 1 and 2, **characterized in that** the door (3) is capable of being moved in the direction of the hollow, door-measuring interface (21).

4. Measuring station according to one of Claims 1 to 3, **characterized in that** the measuring module (5) comprises a module-moving mechanism (15; 26) configured to move the casing-measuring interface (16) between a rest position and a measuring position in the coupled rigid casing (2).

5. Measuring station according to Claims 3 and 4, **characterized in that** the casing-measuring interface (16) and the hollow, door-measuring interface (21) are arranged back-to-back, **in that** the casing-measuring interface (16) is capable of being translated into the coupled rigid casing (2), and **in that** the door (3) is capable of being translated in the direction of the hollow, door-measuring interface (21).

6. Measuring station according to one of the preceding claims, **characterized in that** the sampling line (19) of the particle measuring unit (14) comprises a valve-comprising device (29a, 29b) for selectively switching between the first and second sampling orifices (12, 24).

7. Measuring station according to one of the preceding claims, **characterized in that** the casing-measuring interface (16) comprises at least two injecting nozzles (20) configured to direct a gas jet onto at least two separate locations on the rigid casing (2) coupled to the controlled environment chamber (4), the respective orientations of the injecting nozzles (20) being fixed relative to the coupled rigid casing (2).

8. Measuring station according to one of the preceding claims, **characterized in that** the casing-measuring interface (16) comprises a measuring head (13) protruding from a base of the casing-measuring interface (16).

9. Measuring station according to Claim 8, **characterized in that** said measuring head (13) has a generally parallelepipedal shape, and **in that** each of the five faces of the measuring head (13) protruding from the base of the casing-measuring interface (16) comprises at least one injecting nozzle (20).

10. Measuring station according to either of Claims 7 and 9, **characterized in that** it comprises a processing unit (27) configured to control selective injection of the gas into the injecting nozzles (20, 23).

11. Method for measuring particle contamination of a transport carrier for conveying and storing semiconductor substrates at atmospheric pressure, implemented in a measuring station according to one of Claims 1 to 10, **characterized in that** it comprises:
- a step in which the measuring module (5) couples to the rigid casing (2), thereby defining a first measuring volume (V1) between the casing-measuring interface (16) and the coupled rigid casing (2) in order to measure contamination of the internal walls of the rigid casing (2); and
- a step in which the door (3) couples to the measuring module (5), thereby defining a second measuring volume (V2) between said measuring face (22) and the opposite door (3) in order to measure contamination of the door (3).

12. Measuring method according to Claim 11, **characterized in that** a first interstice is left between the casing-measuring interface (16) and the rigid casing (2) coupled to the casing-measuring interface (16), the gas jet injected into the injecting nozzles (20) being parameterized to generate a leakage gas flow, through the first interstice, directed towards the exterior of the rigid casing (2), and **in that** a second interstice is left between the hollow, door-measuring interface (21) and the door (3) coupled to the hollow, door-measuring interface (21), the gas jet injected into the injecting nozzles (23) being parameterized to generate a leakage gas flow, through the second interstice, directed towards the exterior of the hollow, door-measuring interface (21).
